# NEUE EUROPÄISCHE PATENTSCHRIFT

(11) **EP 2 126 166 B2**
(45) Veröffentlichungstag und Bekanntmachung des Hinweises auf die Entscheidung über den Einspruch: **22.10.2014**
(45) Hinweis auf die Patenterteilung: 14.09.2011
(21) Anmeldenummer: 08716146.9
(22) Anmeldetag: 29.02.2008
(51) Int. Cl.: D01D 5/18, D01F 4/00, D04H 1/42, D04H 1/72, D04H 3/16

(54) **FASER- WIRRGELEGE**
FIBER MATTING
TISSU NON TISSÉ À FIBRES DISPOSÉES DE MANIÈRE ALÉATOIRE

(30) Priorität: 02.03.2007 DE 102007011606
(43) Veröffentlichungstag der Anmeldung: 02.12.2009
(73) Patentinhaber: GELITA AG, 69412 Eberbach (DE); Carl Freudenberg KG, 69469 Weinheim (DE)
(72) Erfinder: AHLERS, Michael, 69412 Eberbach (DE); REIBEL, Denis, F-67850 Herrlisheim (FR); HOFFMANN, Jutta, 69226 Nussloch (DE)
(74) Vertreter: Hoeger, Stellrecht & Partner Patentanwälte
(86) Internationale Anmeldenummer: PCT/EP2008/001623
(87) Internationale Veröffentlichungsnummer: WO 2008/107126

(56) Entgegenhaltungen:
- WO-A1-2006/124856
- WO-A2-2005/100654
- WO-A2-2007/122232
- FR-A1- 2 887 897
- GB-A- 862 428
- JP-A- 3 097 910
- JP-B1- 3 086 822
- US-A- 3 600 482
- US-A1- 2004 011 439

## Beschreibung

Die Erfindung betrifft ein Faser-Wirrgelege, insbesondere auch in Form eines Flächenmaterials oder als Teil eines Flächenmaterials, ein Verfahren zu dessen Herstellung sowie verschiedentliche Verwendungen des Faser-Wirrgeleges.

Die WO 2007/122232 A2 offenbart ein Verfahren zur Herstellung von Gelatinefasern. Bei diesem Verfahren tritt eine wässrige Gelatinelösung aus einer Spinnöffnung aus, während gleichzeitig Druckluft aus Luftdüsen ausgeblasen wird, um die gebildete Faser dünner zu machen oder zu strecken. Aus den auf diese Weise hergestellten Fasern kann ein Wirrgelege gebildet werden.

In der JP 03-086822 B1 wird die Herstellung von Gelatinefasern mittels eines Coazervationsverfahrens beschrieben, bei dem eine wässrige Gelatinelösung in ein organisches Lösungsmittel extrudiert wird. Diese Fasern können anschließend zu einem Wirrgelege verpresst werden.

Die GB 862428 offenbart ein Verfahren und eine Vorrichtung zur Herstellung von Fasern aus verschiedenen natürlichen oder synthetischen makromolekularen Substanzen, u.a. Gelatine, die durch Erwärmen in einen thermoplastischen Zustand überführt und dann versponnen werden.

Die Erfindung zielt insbesondere auf Faser-Wirrgelege wie sie als biologisch abbaubares Material in der Medizintechnik, insbesondere als Implantate oder Trägermaterialien für lebende Zellen (Tissue Engineering), eingesetzt werden können, aber auch auf Faser-Wirrgelege, wie sie in der Lebensmitteltechnologie in einer Vielzahl von Verwendungen, insbesondere als Lebensmittel-Vorprodukt, zum Einsatz kommen können.

Es wird dazu ein neues, erfindungsgemäßes Faser-Wirrgelege vorgeschlagen, welches Fasern aus einem Gelatinematerial umfasst, welche eine Dicke von im Mittel 1 bis 500 µm aufweisen, wobei das Wirrgelege eine Vielzahl von Bereichen aufweist, an denen zwei oder mehr Fasern ohne Phasengrenze ineinander übergehen, wobei das Wirrgelege zwei oder mehr Faserfraktionen mit unterschiedlicher mittlerer Faserdicke umfasst, und wobei das Wirrgelege eine offene Porenstruktur mit einer Luftdurchlässigkeit von 0,5 l/min·cm² oder mehr aufweist, gemessen gemäß DIN 9237. Die Besonderheit der erfindungsgemäßen Faser-Wirrgelege besteht insbesondere darin, dass der Verbund der Fasern in dem Wirrgelege auf die Bereiche zurückzuführen ist, an denen zwei oder mehr Fasern eine Verbindungsstelle ausbilden, an der keine Phasengrenzen erkennbar sind und somit an den Verbindungsstellen durchgängig gleiche Materialverhältnisse beobachtbar sind.

Diese Bereiche sind also nicht durch ein Verkleben oder Verschweißen von aneinander anliegenden Faseroberflächen gebildet, sondern die Besonderheit besteht darin, dass die Faseroberflächen unter Ausbildung der Verbindungsstelle verschwinden.

Insbesondere für die Zwecke der medizinischen Anwendung, und dort insbesondere für die Zwecke des Tissue Engineering, empfehlen sich mittlere Faserdicken im Bereich von 3 bis 200 µm, insbesondere im Bereich von 5 bis 100 µm. Die bevorzugten Faserdicken erlauben insbesondere ein einfaches Besiedeln der Wirrgelege mit lebenden Zellen zur Bildung von Implantaten.

Die erfindungsgemäßen Wirrgelege lassen sich einfach mit der für die Zellbesiedlung erwünschten offenen Porenstrukturen herstellen und bieten eine sehr große spezifische Oberfläche hierfür an.

Gleichzeitig bilden die erfindungsgemäßen Wirrgelege makroskopisch betrachtet ein Trägermaterial, das einer homogenen Zellverteilung nach der Besiedlung förderlich ist. Besonders vorteilhaft ist für das nachfolgende Zellwachstum die interkonnektierende Porenstruktur der erfindungsgemäßen Wirrgelege, die der von porösen Schwammstrukturen überlegen ist.

Auch lassen sich die erfindungsgemäßen Wirrgelege mit einer ausreichenden Formstabilität erzielen, die auch im benetzten Zustand noch ausreichend erhalten bleibt. Dies lässt sich insbesondere durch eine ausreichende Anzahl Einzelfasern mit großem Durchmesser gewährleisten.

Aufgrund der biologischen Verträglichkeit des Gelatinematerials ist auch die Resorption der Wirrgelegeträgerstruktur bei Implantaten gewährleistet.

Das Gelatinematerial in den Fasern ist besonders einfach biologisch abbaubar und zur Steuerung des Abbau-Verhaltens der Fasern des Wirrgeleges ist vorteilhafterweise vorgesehen, dass das Gelatinematerial der Fasern mindestens partiell vernetzt ist. Über den Grad der Vernetzung lässt sich das Abbauverhalten steuern und auch die Festigkeit des Wirrgeleges in feuchtem bis vollständig benetztem oder gequollenem Zustand beeinflussen.

Bei einer besonders bevorzugten Ausführung der vorliegenden Erfindung liegt das Gelatinematerial der Fasern überwiegend amorph vor. Dies hat den Vorteil, dass ein Gelatinematerial der Fasern im amorphen Zustand einfach benetzbar ist. Dies ist insbesondere dann der Fall, wenn das Gelatinematerial der Fasern zu 60 Gew.-% oder mehr in amorphem Zustand vorliegt.

Dies lässt sich auch als Anfangsbenetzbarkeit gegenüber reinem Wasser ausdrücken, die 1 Minute oder weniger betragen sollte. Diese Zeitangabe bemisst sich nach der Zeit, die für die Absorption eines 50 µl großen Tropfens durch ein Wirrgelege mit der Flächendichte von 150 g/m² benötigt wird. Die gute Anfangsbenetzbarkeit drückt sich beispielsweise dadurch aus, dass eine auf eine Wasseroberfläche aufgelegte Probe des Wirrgeleges quasi momentan benetzt wird und unter Aufnahme von Wasser in das Wasser einsinkt.

Zur Kennzeichnung der Struktur des Wirrgeleges, insbesondere dessen Hohlraumstruktur, lässt sich der Kapillarsaugeffekt heranziehen. Dieser sollte bei bevorzugten Wirrgelegen gegenüber reinem Wasser eine Steighöhe des Wassers innerhalb von 120 Sekunden von 15 mm oder mehr erzeugen.

Bei einer weiteren bevorzugten Ausführungsform der Erfindung ist die maximale Wasseraufnahmefähigkeit des Wirrgeleges, die insbesondere durch ein Quellen des, für die Fasern verwendeten Gelatinematerials bedingt oder mit bedingt ist, mindestens das Vierfache von dem Trockengewicht des Wirrgeleges beträgt, d.h. bevorzugt 4 g oder mehr, insbesondere 10 g oder mehr pro Gramm Wirrgelege.

Erfindungsgemäße Wirrgelege weisen bevorzugt eine Oberflächenenergie von 25 mN/m oder weniger auf, insbesondere 10 mN/m oder weniger. Dies erleichtert die Anfangsbenetzung des Wirrgeleges.

Von besonderer Bedeutung der erfindungsgemäßen Wirrgelege ist die Reißfestigkeit, die vorzugsweise bei einem spezifischen Flächengewicht des Wirrgeleges im Bereich von 140 bis 180 g/m² im trockenen Zustand 0,15 N/mm² oder mehr beträgt, wobei des Weiteren bevorzugt eine Reißdehnung im hydratisierten Zustand (Zustand der maximalen Wasseraufnahme durch Quellung) des Wirrgeleges 150 %, insbesondere 200 % oder mehr beträgt.

Solche Wirrgelege lassen sich insbesondere auch bei medizinischen Anwendungen im trockenen Zustand hervorragend handhaben und bieten auch eine ausreichende Festigkeit im hydratisierten, d.h. gequollenen, Zustand, sodass sie sich bei der Verwendung als Implantat-Trägermaterialien sehr einfach an die Gegebenheiten des Körpers an die Implantatstelle anpassen lassen. Insbesondere wird damit auch eine zufriedenstellende Nähfestigkeit zur Fixierung der Implantate erzielt.

Die Wirrgelege der vorliegenden Erfindung weisen eine offene Porenstruktur auf mit einer Luftdurchlässigkeit des Wirrgeleges von 0,5 l/min·cm² oder mehr, wobei die Bestimmung dieses Parameters gemäß DIN 9237 erfolgt. Besonders bevorzugt sind Wirrgelege, bei denen das Gelatinematerial der Fasern in partiell vernetzter Gelform vorliegt, was bedeutet, dass selbst im gequollenen Zustand aufgrund der Vernetzung auch bei Körpertemperatur eines Patienten eine ausreichende Stabilität des Wirrgeleges vorhanden ist, um es zu handhaben, ohne dass das Wirrgelege dabei reißt oder anderweitig beschädigt wird.

Dabei sind insbesondere von Bedeutung solche Wirrgelege, die in hydratisiertem Zustand eine geschlossen-porige fasrige Gelstruktur bilden. Dies bedeutet, dass die Wirrgelege, die im trockenem Zustand durchaus eine offene Porenstruktur aufweisen können und sollen, aufgrund der starken Wasseraufnahme der Gelatine-Anteile und der daraus folgenden Quellung die Offenporigkeit verlieren und dann eine geschlossen-porige fasrige Gelstruktur bilden. Dies ist dann von besonderer Bedeutung, wenn die mit einem Implantat abzudeckenden Gewebebereiche stark bluten und das Implantat gleichzeitig zur Abdeckung von offenen Wunden oder zur Stillung von Blutungen mit verwendet werden soll.

Das Wirrgelege der vorliegenden Erfindung weist insbesondere Fasern aus Gelatinematerial auf, die mit einem Rotor-Spinnverfahren hergestellt sind und mindestens ein Teil der Fasern eine verdrillte Struktur aufweist.

Bevorzugte Gelatinematerialien als Ausgangsmaterial für die Herstellung von Fasern für das erfindungsgemäße Wirrgelege weisen eine Gelstärke von 200 Bloom oder mehr auf.

Weitere bevorzugte Ausführungsformen der vorliegenden Erfindung betreffen Wirrgelege der vorstehend beschriebenen Art, bei denen das Wirrgelege mindestens einen weiteren Typ Fasern umfasst, welche aus einem von dem Gelatinematerial verschiedenen weiteren Material gebildet sind.

Solche weiteren Materialien, aus denen der weitere Typ Fasern gebildet sein kann, sind insbesondere Chitosan, Karrageenan, Alginat, Pektin, Stärke und Stärkederivate, regenerierte Cellulose, oxidierte Cellulose und Cellulose-Derivate, wie z.B. CMC, HPMC, HEC und MC. Des Weiteren sind geeignet synthetische bioverträgliche Polymere, wie z.B. Polymilchsäure und Polylactat-Copolymere, Polyweinsäure, Polycaprolactone, Polyhydroxybuttersäure und Polyethylenterephthalat. Darüber hinaus eignen sich Gelatine-Derivate, wie z.B. Gelatineterephthalat, -carbamylat, -succinat, -dodecylsuccinat, -acrylat (vgl. z.B. EP 0 633 902), sowie Gelatine-Copolymere, wie z.B. Gelatine-Polylactidkonjugat (vgl. DE 102 06 517).

Die Erfindung betrifft des Weiteren ein Flächenmaterial, umfassend ein erfindungsgemäßes Wirrgelege, wie es im Vorstehenden bereits im Einzelnen erläutert wurde. Solche Flächenmaterialien können eine oder mehrere Lagen des erfindungsgemäßen Wirrgeleges umfassen.

Die erfindungsgemäßen Flächenmaterialien umfassen für bestimmte Anwendungszwecke eine sich parallel zu dem Faser-Wirrgelege erstreckende Membran.

Die Membran kann dabei als Trägerschicht für das Wirrgelege dienen, sodass insbesondere sehr geringe Flächengewichte bei dem Wirrgelege realisiert werden können.

Alternativ oder ergänzend kann die Membran eine die Proliferation von Zellen hemmende Sperrschicht bilden, sodass insbesondere bei der Verwendung als Trägermaterial bei Tissue Engineering-Applikationen ein ungestörtes Wachstum der erwünschten oder in das Implantat eingebrachten Zellen möglich ist. In diesem Zusammenhang ist es auch vorteilhaft, wenn die Membran für Zellnährstoffe durchlässig ist.

Die Erfindung betrifft dann weiterhin ein Flächenmaterial der vorstehend beschriebenen Art, wobei das Faser-Wirrgelege mit lebenden Zellen, insbesondere Chondrozyten oder Fibroblasten besiedelt ist.

Bei diesen Applikationen werden insbesondere Faserndurchmesser von im Mittel 3 µm oder mehr verwendet, sodass sich die Zellbesiedlung einfach gestalten lässt. Bevorzugt sind hierbei Porengrößen von im Mittel ca. 100 µm bis ca. 200 µm.

Die Erfindung betrifft des Weiteren die Verwendung des vorstehend beschriebenen Wirrgeleges sowie des ebenfalls vorstehend beschriebenen Flächenmaterials als Zellbesiedlungsmaterial.

Die Erfindung betrifft weiterhin die Verwendung des vorstehend beschriebenen Wirrgeleges sowie des vorstehend beschriebenen Flächenmaterials als medizinische Wundabdeckung.

Die Erfindung betrifft weiterhin die Verwendung des zuvor beschriebenen Wirrgeleges bzw. des zuvor beschriebenen Flächenmaterials als medizinisches Implantat.

Die Erfindung betrifft des Weiteren die Verwendung des zuvor beschriebenen Wirrgeleges als Lebensmittel.

Die erfindungsgemäßen Wirrgelege bzw. die erfindungsgemäßen Flächenmaterialien können auch bei der Herstellung von Depot-Arzneimitteln Verwendung finden. Hierbei kann vorgesehen sein, dass das Gelatinematerial der Fasern einen pharmazeutischen Wirkstoff umfasst.

Optional ergänzend oder alternativ kann das erfindungsgemäße Wirrgelege bzw. das erfindungsgemäße Flächenmaterial als Träger für einen pharmazeutischen Wirkstoff dienen.

Ein bevorzugter pharmazeutischer Wirkstoff, insbesondere bei der Verwendung als Material zur Wundabdeckung ist der Wirkstoff Thrombin.

Ergänzend oder alternativ kann der pharmazeutische Wirkstoff Zellwachstumsfaktoren, insbesondere ein Peptid-Pharmazeutikum, insbesondere Wachstumsmodulatoren, wie z.B. BMP-2, -6, -7, TGF-β, IGF, PDGF, FGF, umfassen.

Die Erfindung betrifft des Weiteren ein Verfahren zur Herstellung von Faser-Wirrgelegen der vorstehend beschriebenen Art, wobei das Verfahren die Schritte umfasst:
(a) Bereitstellen einer ein Gelatinematerial umfassenden wässrigen Spinnlösung;
(b) Erwärmen der Spinnlösung auf eine Spinntemperatur;
(c) Verarbeiten der erwärmten Spinnlösung in einer Spinnvorrichtung mit einem Spinnrotor;
(d) Auffangen der erzeugten Fasern unter Bildung eines Faserwirrgeleges mit einer Vielzahl von Bereichen, an denen zwei oder mehr Fasern ohne Phasengrenze ineinander übergehen;
(e) und optional eine Nachbehandlung des erhaltenen Wirrgeleges durch Zugabe von eigenschaftsverändernden Zusätzen in flüssigem oder gasförmigem Aggregatzustand,
wobei ein Spinnrotor verwendet wird, welcher Spinnöffnungen unterschiedlicher Größe aufweist, sodass Fasern mit unterschiedlicher mittlerer Dicke resultieren.

Das erfindungsgemäße Verfahren arbeitet als Rotationsspinnverfahren, bei dem die von dem Spinnrotor erzeugten Fasern oder Filamente auf einer geeigneten Ablegevorrichtung als Wirrgelege aufgefangen werden.

Als Ablegevorrichtung eignet sich beispielsweise eine Zylinderwand, die konzentrisch zum Spinnrotor angeordnet ist und die eventuell ebenfalls zur Rotation antreibbar ist. Eine weitere Möglichkeit besteht in der horizontalen Ablage der Filamente auf einer Unterlage, z.B. einem Lochblech, das unterhalb des Spinnrotors angeordnet ist.

Über den Abstand zwischen den Austrittsöffnungen des Spinnrotors und der Ablegevorrichtung kann die Flugzeit der Fasern bzw. Filamente vorgegeben werden, die so ausgewählt wird, dass eine ausreichende Verfestigung der in Faserform ausgetragenen Spinnlösung zu ermöglichen, sodass die Faserform beim Auftreffen auf die Ablegevorrichtung erhalten bleibt.

Dies wird zum einen durch das Abkühlen der Faser- bzw. Filamentmaterialien während der Flugzeit unterstützt, zum anderen durch die Gelbildung der Gelatine und des Weiteren durch ein Verdampfen von Wasser, bzw. der Lösungsmittel.

Die von dem Spinnrotor erzeugten Fasern bzw. Filamente lassen sich einfach in einem Zustand auffangen, indem sich in einer Vielzahl von Bereichen des Wirrgeleges Verbindungsstellen zwischen zwei oder mehreren Fasern bilden, an denen diese ohne Phasengrenze in einander übergehen.

In dem optionalen Nachbehandlungsschritt (d) lässt sich das erfindungsgemäße Wirrgelege in einer Vielzahl von Eigenschaften an spezifische Applikationen anpassen.

Durch Vernetzung des Gelatinematerials können die mechanischen und insbesondere chemischen Eigenschaften modifiziert werden. Beispielsweise lassen sich über den Vernetzungsgrad des Gelatinematerials die Resorptionseigenschaften für medizinische Anwendungszwecke vorgeben.

Das regelmäßig hochflexible Wirrgelege der vorliegenden Erfindung lässt sich in Nachbehandlungsschritten versteifen, beispielsweise um die Formstabilität zu verbessern und das Einbringen in einen Zielbereich zu erleichtern.

Die erfindungsgemäßen Wirrgelege lassen sich in Nachbehandlungsschritten mit flüssigen Medien tränken und/oder beschichten. Dafür kommen insbesondere andere biologisch abbaubare Polymermaterialien oder auch wachsartige Materialien in Frage.

Mittels des vorstehend beschriebenen erfindungsgemäßen Verfahrens lassen sich insbesondere auf einfache Weise die Faser-Wirrgelege der vorliegenden Erfindung erzeugen, bei denen eine Faserdicke im Mittel von 1 bis 500 µm erzeugt wird, und wobei des Weiteren die für die Erfindung charakteristischen Bereiche gebildet werden, an denen zwei oder mehr Fasern ohne Phasengrenze miteinander verbunden oder quasi verschmolzen sind. Bevorzugt wird bei dem erfindungsgemäßen Verfahren eine Spinnlösung verwendet, bei der der Anteil an Gelatine im Bereich von ca. 10 bis ca. 40 Gew.-% beträgt.

Die Gelfestigkeit der Gelatine beträgt dabei bevorzugt ca. 120 bis ca. 300 Bloom.

Die Spinnlösung wird vorzugsweise auf eine Spinntemperatur im Bereich von ca. 40 °C oder mehr, insbesondere im Bereich von ca. 60 bis ca. 97 °C erwärmt. Diese Temperaturen erlauben insbesondere ein einfaches Bilden der charakteristischen Bereiche der Faser-Wirrgelege, an denen zwei oder mehr Fasern ohne Phasengrenzen miteinander verbunden sind bzw. ineinander übergehen.

Bevorzugt wird die Spinnlösung vor der Verarbeitung in Schritt (c) entgast, sodass lange Fasern mit einer sehr homogenen Faserdicke in dem Wirrgelege erhalten wird.

Das Entgasen wird vorzugsweise mittels Ultraschall durchgeführt.

Zur Erzeugung von partiell vernetzten Gelatinematerialien in den Fasern wird der Spinnlösung bevorzugt bereits ein Vernetzungsmittel zugefügt. Eine Vernetzung lässt sich allerdings auch und zusätzlich bei den bereits versponnenen Fasern durch in Kontakt bringen mit einem Vernetzungsmittel, sei es gasförmig oder in Lösung, bewirken.

Eine besonders zuverlässige Durchführung des erfindungsgemäßen Verfahrens wird dann möglich, wenn der Rotor auf eine Temperatur von ca. 100 bis ca. 140 °C erwärmt wird. Diese Temperatur ist besonders geeignet um die Gelatinematerialien umfassenden wässrigen Spinnlösungen im Rotationsspinnverfahren zu verarbeiten.

Am bereits fertig vorliegenden Wirrgelege wird vorzugsweise eine weitere Vernetzung vorgenommen, die dann den endgültigen Vernetzungsgrad des Gelatinematerials in dem Wirrgelege bestimmt und damit dessen biologische Abbaurate.

Für die Vernetzung stehen verschiedene Verfahren zur Verfügung, wobei enzymatische Verfahren, die Verwendung von Komplexbildnern oder chemische Verfahren bevorzugt sind.

Bei der chemischen Vernetzung wird die Vernetzung mittels einem oder mehreren Reaktanden durchgeführt, insbesondere mit Aldehyden, ausgewählt aus Formaldehyd und Dialdehyden, Isocyanaten, Diisocyanaten, Carbodiimiden, Alkyldihalogeniden, und hydrophilen Di- und Trioxiranen wie z.B. 1,4 Butandiol-diglycidether und Glycerin-triglycidether.

Insbesondere bei der medizinischen Anwendung empfiehlt es sich, nach dem Vernetzen überschüssiges Vernetzungsmittel aus dem Wirrgelege bzw. dem Flächenmaterial zu entfernen.

Wie zuvor beschrieben ist es bevorzugt, der Spinnlösung bereits ein Vernetzungsmittel zuzugeben, und dann an dem fertigen Wirrgelege, sozusagen in einer zweiten Stufe, eine weitere Vernetzung bis zum gewünschten Vernetzungsgrad durchzuführen.

Die Wirrgelege der vorliegenden Erfindung können insbesondere als extrem flexible Flächenmaterialien hergestellt werden, sind dabei elastisch und sehr gut modellierbar. Darüber hinaus können die Wirrgelege als völlig offene Strukturen betrachtet werden gegenüber Schwammstrukturen, die ebenfalls schon als Trägermaterial beim Tissue Engineering verwendet worden sind und ebenfalls porös sind, jedoch Zellwände aufweisen.

Es lassen sich dabei sehr kleine Filamentdicken, insbesondere mit dem erfindungsgemäß vorgeschlagenen Spinnrotor-Spinnverfahren herstellen, wobei die Gelatine während des gesamten Spinnvorganges nur sehr kurzzeitig höheren Temperaturen ausgesetzt werden muss, d.h. die Temperaturbelastung des Gelatinematerials kann zeitlich sehr stark beschränkt werden und führt zu Fasern aus Gelatinematerial, welches im Wesentlichen in seinem Molekulargewichtspektrum dem Ausgangsgelatinematerial entspricht.

Im Rahmen der vorliegenden Erfindung können Wirrgelege einen Anteil an Fasern aufweisen, deren mittlere Faserdicke sie von den anderen Fasern unterscheidet. Insbesondere können sie eine arößere mittlere Faserdicke aufweisen. Durch die Verwendung von zwei oder mehr Faserfraktionen im Wirrgelege, die sich durch ihre mittlere Faserdicke unterscheiden, können gezielt dessen mechanische Festigkeitswerte beeinflusst werden.

Wiederum alternativ oder ergänzend können zwei oder mehr Lagen an Wirrgelegen zu einem Flächenmaterial kombiniert werden, wobei die einzelnen Lagen Fasern unterschiedlicher mittlerer Faserdicke aufweisen können. Selbstverständlich sind auch bei diesen Flächenmaterialien die Verwendung von Wirrgelege-Lagen mit Fasern von im Wesentlichen einheitlicher mittlerer Faserdicke neben Wirrgelege-Lagen mit mehreren Faserfraktionen unterschiedlicher mittlerer Faserdicke möglich.

Wirrgelege mit Faserfraktionen unterschiedlicher mittlerer Faserdicke, z.B. ca. 7 µm neben ca. 25 µm, lassen sich mit dem erfindungsgemäßen Verfahren dadurch realisieren, dass man einen Spinnrotor verwendet, in dem Spinndüsen mit unterschiedlich großen Düsenöffnungen beim Spinnvorgang vorgesehen sind.

Bei einer Verwendung des erfindungsgemäßen Wirrgeleges als Trägermaterial für lebende Zellen, weist das Wirrgelege einen großen Vorteil gegenüber Schwammstrukturen oder Gewebestrukturen dadurch auf, dass sehr unterschiedliche Hohlräume für das Einlagern der Zellen angeboten werden, sodass die Zellen die für sie idealen Einlagerungsstellen finden können. Dies gilt bereits bei Wirrgelegen, die eine einheitliche mittlere Faserdicke aufweisen.

Diese und weitere Vorteile der vorliegenden Erfindung werden im Folgenden anhand der Zeichnung sowie von Beispielen noch näher erläutert.

Es zeigen im Einzelnen:
- Figur 1:: eine schematische Darstellung einer Vorrichtung zur Durch- führung des erfindungsgemäßen Verfahrens;
- Figur 2a bis c:: mikroskopische Aufnahmen eines erfindungsgemäßen Wirr- geleges in unterschiedlicher Vergrößerung;
- Figur 3:: ein Steighöhen/Zeit-Diagramm für verschiedene Materiali- en;
- Figur 4a bis c:: eine schematische Darstellung einer Vorrichtung zur Ermitt- lung der in Figur 3 dargestellten Steighöhen; und
- Figur 5a und b:: Zug/Dehnungsergebnisse von erfindungsgemäßen und her- kömmlichen Zellträgermaterialien.

### Referenz-Beispiel 1:

### Herstellung eines Faser-Wirrgeleges

Es wird eine 20 %ige wässrige Lösung einer Schweineschwartengelatine (300 Bloom) durch Mischen von 20 g Gelatine und 80 ml destilliertem Wasser bei Raumtemperatur hergestellt. Nach einem Quellen der Gelatine während ca. 60 Minuten wird die Lösung für eine Stunde auf 60 °C erhitzt und danach mit Ultraschall entgast.

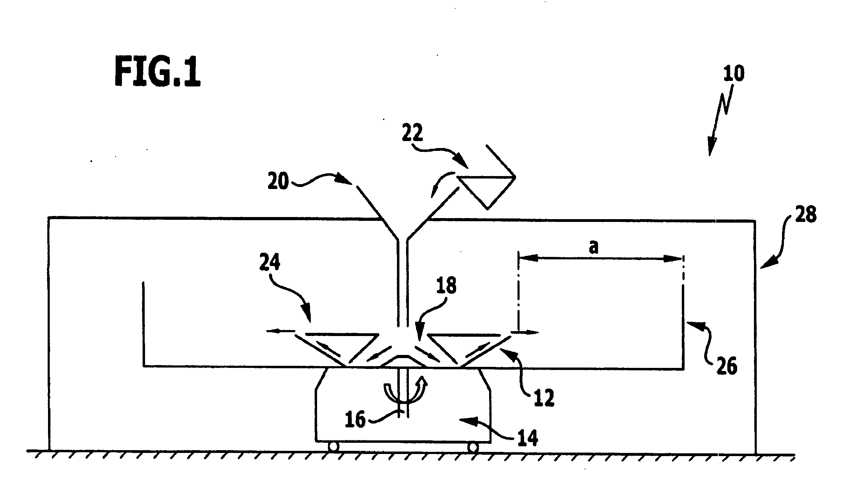

Diese Lösung wird dann mit einer Spinnvorrichtung 10 verarbeitet wie sie schematisch in Figur 1 gezeigt ist. Ebenso geeignet sind Spinnvorrichtungen wie sie in der DE 10 2005 048 939 A1 beschrieben sind, auf die vollinhaltlich Bezug genommen wird.

Die Spinnvorrichtung 10 umfasst einen Spinnrotor 12, der von einem Antriebsaggregat 14 um eine vertikale Drehachse 16 in Rotation versetzt werden kann.

Der Spinnrotor 12 weist einen Behälter 18 zur Aufnahme der wässrigen Gelatinespinnlösung auf, die während des Spinnvorgangs kontinuierlich über einen Trichter 20 von einem Zulaufkanal 22 aus befüllt werden kann.

Der Behälter 18 weist an seinem äußeren Umfang eine Mehrzahl an Öffnungen 24 auf, über die durch Zentrifugalkraft die Spinnlösung in Filamentform ausgetragen wird.

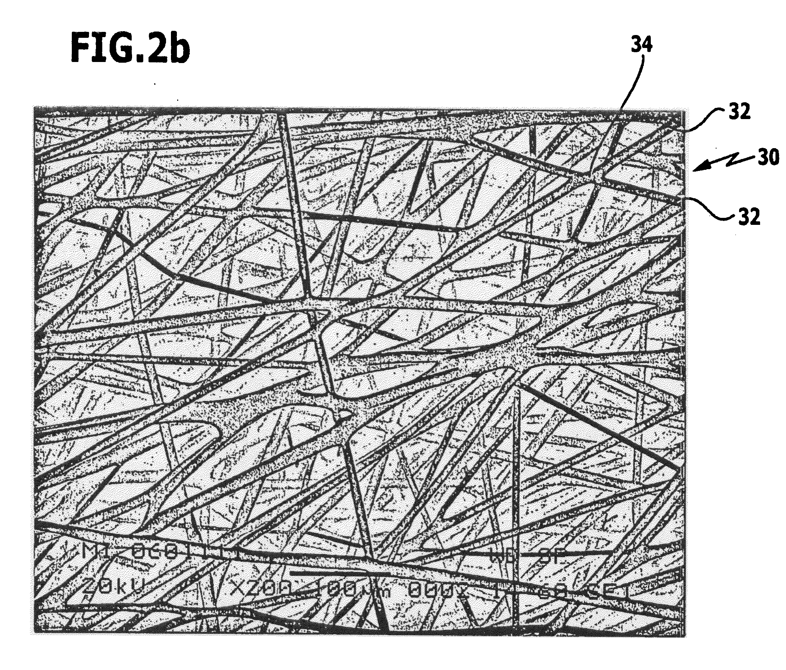

In einem vorgegebenen Abstand a zu den Öffnungen 24 ist eine Ablegevorrichtung 26 in Form einer Zylinderwandung vorgesehen, die die zu Filamenten bzw. Fasern geformte Spinnlösung auffängt. Aufgrund der über den Abstand a vorgegebenen Flugzeit bei einer bestimmten Rotationsgeschwindigkeit des Spinnrotors 12 wird die die Filamente bzw. Fasern bildende Spinnlösung soweit verfestigt, dass die Filamentform beim Auftreffen auf die Ablegevorrichtung 26 im Wesentlichen erhalten bleibt, andererseits sich noch die Bereiche ausbilden können, in denen zwei oder mehrere Fasern bzw. Filamente quasi miteinander verschmelzen und Verbindungsstellen schaffen, bei denen die Phasengrenzen der aneinanderliegenden Faserabschnitte aufgehoben sind (vgl. insbesondere Figur 2b).

Der Spinnrotor 12 mitsamt dem Antriebsaggregat 14 und der Ablegevorrichtung 26 sind in einem Gehäuse 28 angeordnet, welches einen Spinnraum von der Umgebung abgrenzt.

Im vorliegenden Beispiel wird der Spinnrotor 12 bei einer Rotationsdrehzahl von 2.000 bis 3.000 U/min betrieben. Der Rotor 12 wird auf eine Temperatur von 130 °C erhitzt. Die Gelatinelösung wird auf 95°C aufgeheizt und dem Rotor 12 zugeführt, sodass eine kontinuierliche Filamenterzeugung durchgeführt werden kann. Mittels einer Absaugung werden die Filamente als Vlies auf der Ablegevorrichtung 26 abgelegt. Der Abstand a beträgt ca. 20 cm und definiert damit eine Flugzeit von ca. 0,01 m sec.

Über die Größe der Öffnungen 24 des Behälters 18 des Spinnrotors 12, der Rotationsgeschwindigkeit des Spinnrotors 12 sowie der Konzentration an Gelatine in der Spinnlösung lässt sich der mittlere Durchmesser der erhaltenen Filamente bzw. Fasern beeinflussen. Im vorliegenden Beispiel beträgt der Durchmesser der Öffnungen 24 ca. 0,9 mm.

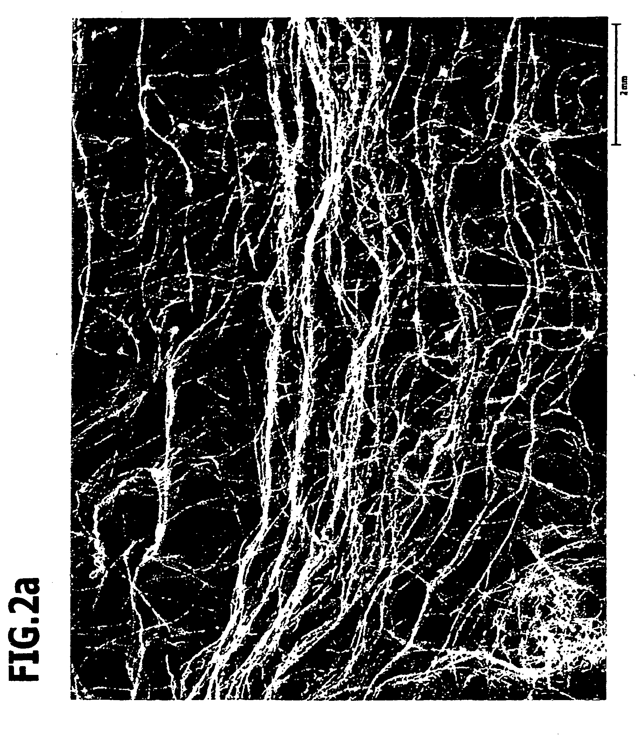

Bei dem vorgenannten Beispiel werden Filamente mit einer Filamentdicke im Bereich von 2,5 bis 14 µm (mittlere Faserdicke 7,5 µm ± 2,6 µm) erhalten. Ein beispielhaftes in dem erfindungsgemäßen Verfahren erhältliches Wirrgelege ist in unterschiedlicher Vergrößerung in den Figuren 2a bis c dargestellt.

Das relativ lockere Wirrgelege wie in Figur 2a gezeigt kann selbstverständlich mit höherer Filament- oder Faserdichte erhalten werden, jedoch können auch Wirrgelege mit der Dichte wie in Figur 2a gezeigt mehrfach aufeinander gelegt zu einem selbsttragendem Flächengebilde in Form eines Vlieses verbunden werden, oder aber auf Trägermaterialien wie z.B. Membranen oder Folien aufgelegt werden.

Figur 2b zeigt in einer Rasterelektronenmikroskopaufnahme die mit dem erfindungsgemäßen Verfahren erhältlichen und die erfindungsgemäßen Wirrgelege 30 mit einer Vielzahl an Fasern 32 aus einem Gelatinematerial und insbesondere den die Erfindung auszeichnenden Bereichen 34, in denen zwei oder mehr Fasern 32 miteinander ohne Phasengrenze verbunden sind.

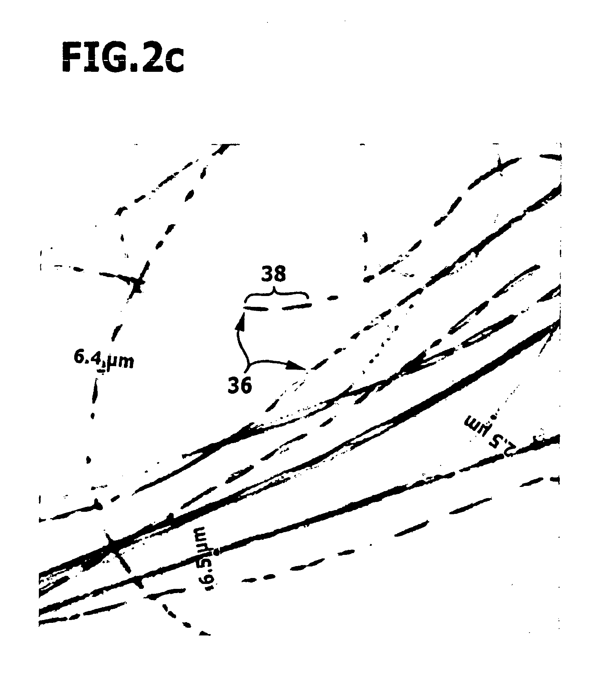

In Figur 2c ist in einer lichtmikroskopischen Aufnahme in polarisiertem Licht der Effekt der Verdrillung der einzelnen Filamente 36 sichtbar gemacht, wobei die Verdrillungsabschnitte durch Hell-Dunkel-Bereiche 38 visualisiert sind.

### Referenz-Beispiel 2:

### Herstellung eines Zellträgermaterials

Aus dem in Beispiel 1 gewonnen Wirrgelege werden vorgegebene Flächenstücke ausgestanzt und lagenweise aufeinandergelegt bis ein Vlies mit einem gewünschten Flächengewicht, beispielsweise im Bereich von ca. 20 bis ca. 500 g/m² erzielt wird.

Im vorliegenden Beispiel wird ein mehrlagiges Vlies gebildet mit einem Flächengewicht von 150 g/m² hergestellt und anschließend mit Hilfe von gasförmigen Formaldehyd partiell vernetzt. Die Vernetzungsbedingungen im Einzelnen waren die Folgenden:

Das Wirrgelege wird in einer Gasatmosphäre über einer 10 Gew.-%igen Formaldehyd-Lösung für ca. 17 h inkubiert. Danach wird das Wirrgelege 48 h bei ca. 50 °C und 70 % relativer Feuchte in einem Klimaschrank getempert. Hierbei wird die Vernetzungsreaktion vervollständigt und der überschüssige Anteil an nicht verbrauchtem Formaldehyd (Vernetzer) entfernt.

Aus solchermaßen hergestellten Vliesen wurden Proben ausgestanzt und in ihren Wasseraufnahmeeigenschaften sowie mechanischen Eigenschaften mit herkömmlichen Zellträgermaterialien in Form von porösen Gelatineschwämmen sowie einem Material aus oxidierter Zellulose verglichen.

Die Probenbreite betrug jeweils 1 cm.

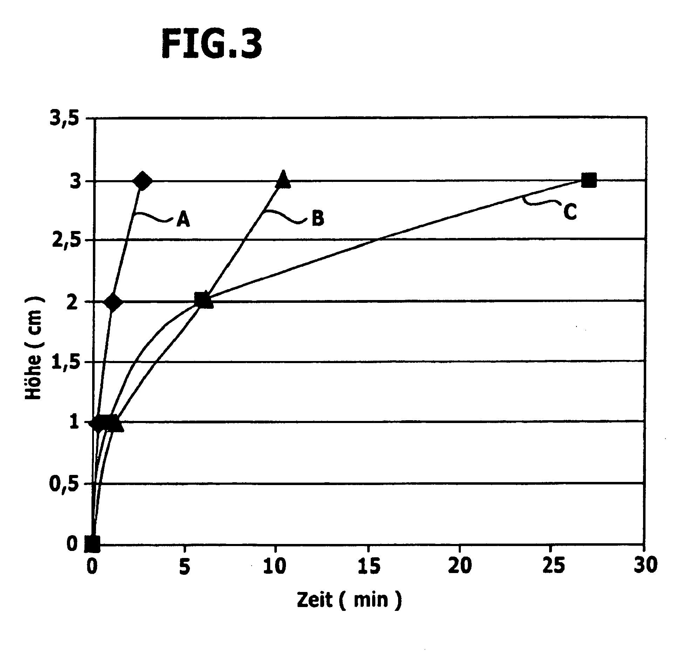

Figur 3 zeigt die Steighöhe von reinem Wasser über die Zeit aufgetragen für diese drei Materialien, wobei die mit dem Buchstaben A bezeichnete Kurve dem erfindungsgemäßen Vlies als mehrlagiges Wirrgelege entspricht, die Kurve B einem herkömmlichen Gelatineschwamm und die Kurve C dem herkömmlichen, kommerziell erhältlichen Zellulosematerial.

Aus dem Vergleich der Wasseraufnahme über die Zeiteinheit wird deutlich, dass Gelatinematerialien den Zellulosematerialien wie in dem der Probe C verwendet, deutlich überlegen sind.

Die Probe des Vlieses aus dem erfindungsgemäßen Wirrgelege gemäß Kurve A ist dann dem Gelatinematerial in Schwammform (Kurve B), wie dies aus der Figur 3 ersichtlich ist, nochmals in der Wasseraufnahmefähigkeit pro Zeiteinheit deutlich überlegen.

Der praktische Vorteil dieser deutlich erhöhten Wasseraufnahmegeschwindigkeit liegt darin, dass Flüssigkeiten, wie z.B. Blut schneller und stärker aufgesaugt werden und im Falle von zu versorgenden Wunden zu einer verbesserten Blutstillung führen.

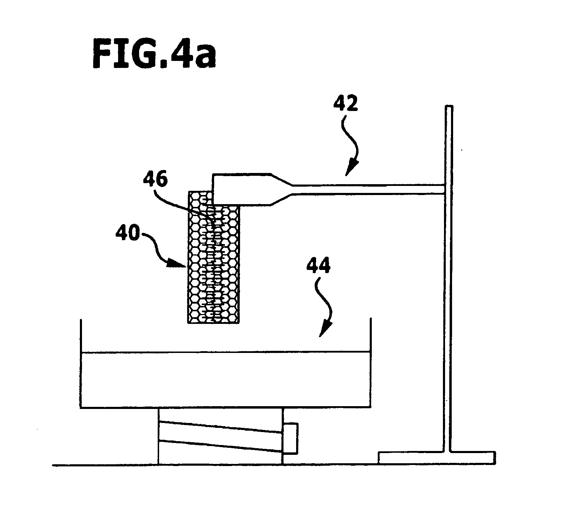

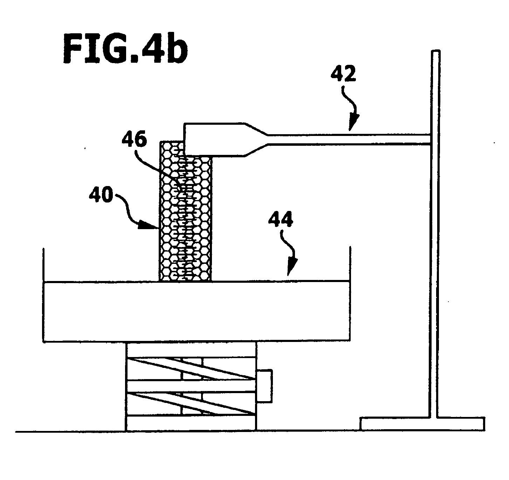

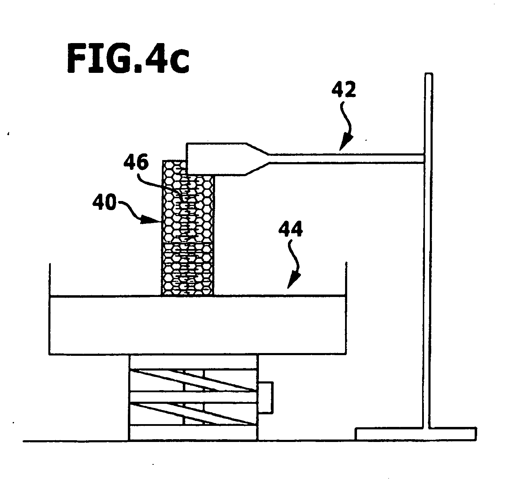

In der Figur 4a bis c ist das Prinzip der Messung für die Steighöhe pro Zeiteinheit schematisch dargestellt. Die vorbereitete Probe 40 wird über einen Halter 42 frei nach unten hängend eingespannt, und über einem Becken 44 mit temperiertem Wasser (25 °C) platziert. Zu Beginn der Messung wird der Behälter mit dem Wasser soweit nach oben gefahren, dass die Probe 2 mm tief in den Wasservorrat eintaucht. Danach wird zeitabhängig die über Kapillarkräfte erzeugte Steighöhe registriert und dann in dem Diagramm gemäß Figur 3 eingetragen. Ein auf der Probe 40 aufgetragener Maßstab 46 erleichtert das Ablesen der Steighöhe.

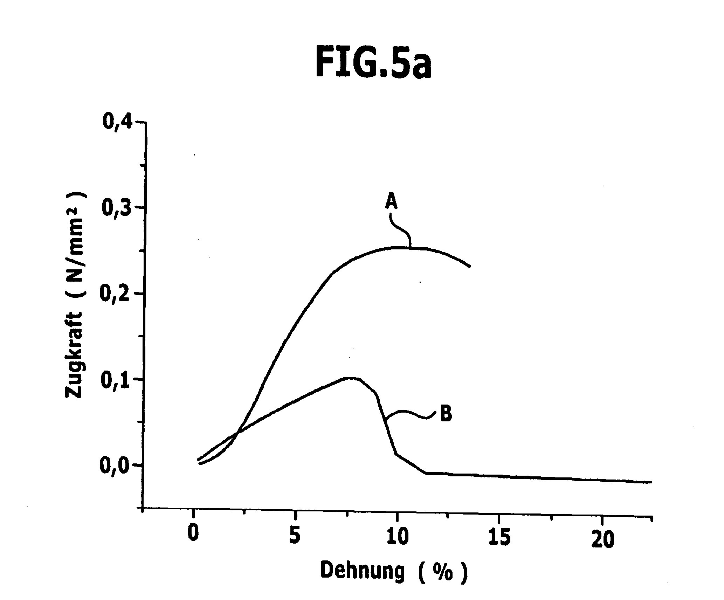

An den zuvor beschriebenen Proben mit einer Breite von 15 mm und einer Dicke von ca. 1 mm wurden auch Zug/Dehnungsmessungen durchgeführt und zwar im trockenen Zustand (Figur 5a). Verglichen wurden hierbei nur die beiden Gelatine basierenden Proben, d.h. einmal das erfindungsgemäß hergestellte Vlies und zum anderen die herkömmliche Schwammprobe mit den gleichen Abmessungen.

Aus der Figur 5a ist ersichtlich, dass das Gelatinevlies gemäß der vorliegenden Erfindung gegenüber dem Gelatineschwamm im trockenen Zustand (Wassergehalt ca. 10 Gew.-%) eine weitaus höhere spezifische Zugfestigkeit aufweist und darüber hinaus auch schon im trockenen Zustand eine erheblich größere Dehnung zulässt. Während die Zug/Dehnungskurve für die Gelatineschwammprobe (Kurve B) bereits nach einer Dehnung von ca. 7 bis 8 % abbricht, d.h. die Probe reißt, lässt sich die erfindungsgemäße Vliesprobe um ca. 17 % dehnen, bevor ein Reißen der Probe beobachtet wird. Dabei wird außerdem noch eine deutlich höhere Zugfestigkeit gegenüber der Schwammprobe festgestellt.

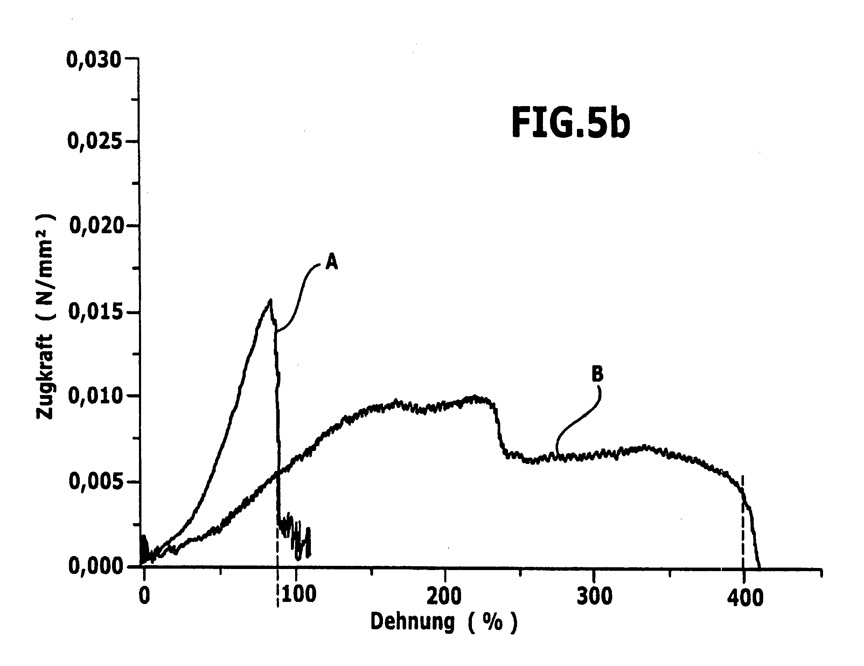

Noch viel größere und bedeutsamere Unterschiede erhält man im vollhydratisierten Zustand der Proben (Figur 5b), d.h. in einem Zustand, in dem das vernetzte Gelatinematerial des Schwammes bzw. des erfindungsgemäßen Vlieses vollständig gequollen ist. Der Wassergehalt beträgt hier mehr als 100 Gew.-% bezogen auf das Gelatinematerial.

Für den Vergleich wurde ein Standardschwamm in den Maßen 80x50x10 mm sowie das erfindungsgemäße Vlies in den Maßen 80x50x1 mm verwendet. Der Schwamm weist ein Trockenflächengewicht von 120 g/m² auf, das Vlies eines von 180 g/m².

Hier wird bei der Schwammprobe nach einer Dehnung von knapp 75 % ein Reißen der Probe beobachtet (Kurve B), während sich die erfindungsgemäße Vliesprobe auf 400 % dehnen lässt (Kurve A), bevor diese endgültig reißt. Auch im hydratisierten Zustand erreicht das Vlies (mit 2,6 N Zugkraft) eine höhere Festigkeit als der Schwamm.

Dies ist von ganz besonderer Bedeutung bei der Verwendung der Vliesmaterialien als Träger für Zellimplantate, da dies dem behandelnden Arzt die Möglichkeit lässt, das Zellimplantat nahezu beliebig zu verformen, zu dehnen und an die Gegebenheiten der zur versorgenden Wunde des Patienten anzupassen.

### Referenz-Beispiel 3:

### Herstellung von zuckerfreier Zuckerwatte

Analog zu Beispiel 1 wird eine 20 Gew.-%ige wässrige Spinnlösung mit folgender Zusammensetzung hergestellt:
15 g Gelatine Typ A, 260 Bloom, Speisequalität
15 g Gelatinehydrolysat Typ A, mittleres Molekulargewicht 3 kD
70 g Wasser

Farbstoffe (z.B. Himbeere) und Aromen (z.B. Vanille-Cola) können nach Herstellerangaben zudosiert werden.

Die Spinnlösung wird auf 70 °C erwärmt und im Spinnrotor versponnen.

Das aufgefangene Produkt hat die Konsistenz und Sensorik von Zuckerwatte.

## Patentansprüche

1. Faser-Wirrgelege, umfassend Fasern aus einem Gelatinematerial, wobei die Dicke der Fasern im Mittel 1 bis 500 µm beträgt und wobei das Wirrgelege eine Vielzahl von Bereichen aufweist, an denen zwei oder mehr Fasern ohne Phasengrenze ineinander übergehen, **dadurch gekennzeichnet, dass** das Wirrgelege zwei oder mehr Faserfraktionen mit unterschiedlicher mittlerer Faserdicke umfasst, und dass das Wirrgelege eine offene Porenstruktur mit einer Luftdurchlässigkeit von 0,5 l/min·cm² oder mehr aufweist, gemessen gemäß DIN 9237.

2. Wirrgelege nach Anspruch 1, **dadurch gekennzeichnet, dass** das Gelatinematerial der Fasern zu 60 Gew.-% oder mehr amorph vorliegt.

3. Wirrgelege nach einem der voranstehenden Ansprüche, **dadurch gekennzeichnet, dass** das Wirrgelege mindestens einen weiteren Typ Fasern umfasst, welche von einem von dem Gelatinematerial verschiedenen weiteren Material gebildet sind.

4. Wirrgelege nach Anspruch 3, **dadurch gekennzeichnet, dass** das weitere Material ausgewählt ist aus Chitosan, Carrageenan, Alginat, Pektin, Stärke und Stärkederivate, regenerierte Cellulose, oxidierte Cellulose und Cellulose-Derivate, wie z.B. CMC, HPMC, HEC und MC, Gelatine-Derivate, insbesondere Gelatineterephthalat, -carbamat, -succinat, -dodecylsuccinat, -acrylat, sowie Gelatine-Copolymere, wie z.B. Gelatine-Polylactidkonjugat.

5. Flächenmaterial mit einer oder mehreren Lagen, wobei mindestens eine der Lagen ein Wirrgelege nach einem der voranstehenden Ansprüche umfasst.

6. Flächenmaterial nach Anspruch 5, **dadurch gekennzeichnet, dass** eine erste Lage ein Wirrgelege Fasern mit einer ersten mittleren Dicke und eine zweite Lage ein Wirrgelege Fasern mit einer zweiten mittleren Dicke umfasst, wobei die zweite mittlere Dicke größer ist als die erste mittlere Dicke.

7. Flächenmaterial nach Anspruch 5 oder 6, **dadurch gekennzeichnet, dass** es eine sich parallel zu dem Faser-Wirrgelege erstreckende Membran umfasst.

8. Flächenmaterial nach einem der Ansprüche 5 bis 7, **dadurch gekennzeichnet, dass** das Faser-Wirrgelege mit lebenden Zellen, insbesondere Chondrozyten oder Fibroblasten besiedelt ist.

9. Verwendung eines Wirrgeleges gemäß einem der Ansprüche 1 bis 4 oder eines Flächenmaterials nach einem der Ansprüche 5 bis 7 als Zellbesiedelungsmaterial.

10. Verwendung eines Wirrgeleges nach einem der Ansprüche 1 bis 4 als Lebensmittelvorprodukt.

11. Verwendung eines Wirrgeleges nach einem der Ansprüche 1 bis 4 oder eines Flächenmaterials nach einem der Ansprüche 5 bis 7 bei der Herstellung eines Depotarzneimittels.

12. Verwendung eines Wirrgeleges nach einem der Ansprüche 1 bis 4 oder eines Flächenmaterials nach einem der Ansprüche 5 bis 7 als Träger für einen pharmazeutischen Wirkstoff.

13. Verfahren zur Herstellung von Faser-Wirrgelegen nach einem der Ansprüche 1 bis 4, umfassend die Schritte
(a) Bereitstellen einer ein Gelatinematerial umfassenden wässrigen Spinnlösung;
(b) Erwärmen der Spinnlösung auf eine Spinntemperatur;
(c) Verarbeiten der erwärmten Spinnlösung in einer Spinnvorrichtung mit einem Spinnrotor, wobei Fasern aus dem Gelatinematerial erzeugt werden; und
(d) Auffangen der erzeugten Fasern unter Bildung eines Faser-Wirrgeleges mit einer Vielzahl von Bereichen, an denen zwei oder mehr Fasern ohne Phasengrenze ineinander übergehen,
**dadurch gekennzeichnet, dass** ein Spinnrotor verwendet wird, welcher Spinnöffnungen unterschiedlicher Größe aufweist, sodass Fasern mit unterschiedlicher mittlerer Dicke resultieren.

14. Verfahren nach Anspruch 13, **dadurch gekennzeichnet, dass** die Spinnlösung einen Anteil an Gelatine im Bereich von ca. 10 bis ca. 40 Gew.-% umfasst.

## Claims

1. A non-woven fiber fabric comprising fibers of a gelatin material, wherein the thickness of the fibers is on average 1 to 500 µm and wherein the non-woven fiber fabric has a plurality of areas where two or more fibers merge into one another without any phase boundary, **characterized in that** the non-woven fiber fabric contains two or more fiber fractions with a different average fiber thickness, and that the non-woven fiber fabric exhibits an open-pore structure with an air permeability of 0.5 l/min·cm² or more, measured according to the DIN 9237 standard.

2. The non-woven fiber fabric of claim 1, **characterized in that** the gelatin material of the fibers is amorphous to 60% by weight of more.

3. The non-woven fiber fabric of any of the preceding claims, **characterized in that** the non-woven fiber fabric comprises at least one additional type of fibers formed from an additional material different to the gelatin material.

4. The non-woven fiber fabric of claim 3, **characterized in that** the additional material is selected from chitosan, carrageenan, alginate, pectin, starch and starch derivatives, regenerated cellulose, oxidized cellulose and cellulose derivatives, such as, for example, CMC, HPMC, HEC and MC, gelatin derivatives, in particular, gelatin terephthalate, gelatin carbamate, gelatin succinate, gelatin dodecyl succinate, gelatin acrylate as well as gelatin copolymers, such as, for example, gelatin polylactide conjugate.

5. A sheet material with one or more layers, wherein at least one of the layers contains a non-woven fiber fabric of any of the preceding claims.

6. The sheet material of claim 5, **characterized in that** a first layer contains a non-woven fiber fabric of fibers with a first average thickness and a second layer contains a non-woven fiber fabric of fibers with a second average thickness, wherein the second average thickness is greater than the first average thickness.

7. The sheet material of claim 5 or claim 6, **characterized in that** it contains a membrane extending parallel to the non-woven fiber fabric.

8. The sheet material of any of claims 5 to 7, **characterized in that** the non-woven fiber fabric is colonized by living cells, in particular, chondrocytes or fibroblasts.

9. Use of a non-woven fiber fabric in accordance with any of claims 1 to 4 or a sheet material of any of claims 5 to 7 as a cell colonization material.

10. Use of a non-woven fiber fabric of any of claims 1 to 4 as a food precursor.

11. Use of a non-woven fiber fabric of any of claims 1 to 4 or a sheet material of any of claims 5 to 7 for the production of a depot medicament.

12. Use of a non-woven fiber fabric of any of claims 1 to 4 or a sheet material of any of claims 5 to 7 as a carrier for a pharmaceutical substance.

13. A method for producing the non-woven fiber fabrics of any of claims 1 to 4, including the steps of
(a) providing an aqueous spinning solution containing a gelatin material;
(b) heating the spinning solution to a spinning temperature;
(c) processing the heated spinning solution in a spinning device with a spinning rotor, wherein fibers of the gelatin material are produced; and
(d) collecting the produced fibers so as to form of a non-woven fiber fabric with a plurality of areas where two or more fibers merge into one another without any phase boundary,
**characterized in that** a spinning rotor is used having spinning openings of different sizes so that fibers with different average thicknesses result.

14. The method of claim 13, **characterized in that** the spinning solution contains a proportion of gelatin in the range of approximately 10 to approximately 40 % by weight.

## Revendications

1. Textile en fibres non-tissé, comprenant des fibres en matériau à base de gélatine, dans lequel l'épaisseur des fibres est en moyenne de 1 à 500 µm, et le textile non-tissé comprend une pluralité de zones au niveau desquelles deux ou plusieurs fibres se transforment les unes dans les autres sans limite de phase,
**caractérisé en ce que** le textile non-tissé comprend deux ou plusieurs fractions fibreuses avec des épaisseurs de fibres moyennes différentes, et que le textile non-tissé présente une structure à pores ouverts avec une perméabilité à l'air de 0,5 l/min·cm² ou plus, mesuré selon la norme DIN 9237.

2. Textile non-tissé selon la revendication 1, **caractérisé en ce que** le matériau à base de gélatine des fibres se présente sous forme amorphe à raison de 60 % en poids ou plus.

3. Textile non-tissé selon l'une des revendications précédentes, **caractérisé en ce que** le textile non-tissé comprend au moins un autre type de fibres qui sont formées par un autre matériau différent d'un matériau à base de gélatine.

4. Textile non-tissé selon la revendication 3, **caractérisé en ce que** l'autre matériau est choisi parmi chitosane, carraghénane, alginate, pectine, amidon et dérivés d'amidon, cellulose régénérée, cellulose oxydée et dérivés de cellulose comme par exemple CMC, HPMC, HEC, et MC, dérivés de gélatine, en particulier téréphtalate-, carbamate-, succinate-, dodécylsuccinate-, acrylate- de gélatine, ainsi que copolymères de gélatine, comme par exemple un conjugué polylactide de gélatine.

5. Matériau surfacique comprenant une ou plusieurs couches, dans lequel l'une au moins des couches comprend un textile non-tissé selon l'une des revendications précédentes.

6. Matériau surfacique selon la revendication 5, **caractérisée en ce qu'**une première couche comprend un textile non-tissé de fibres présentant une première épaisseur moyenne et une seconde couche comprend un textile non-tissé de fibres présentant une seconde épaisseur moyenne, la seconde épaisseur moyenne étant supérieure à la première épaisseur moyenne.

7. Matériau surfacique selon la revendication 5 ou 6, **caractérisé en ce qu'**il comprend une membrane s'étendant parallèlement au textile en fibres non-tissé.

8. Matériau surfacique selon l'une des revendications 5 à 7, **caractérisé en ce que** le textile en fibres non-tissé est peuplé de cellules vivantes, en particulier de chondrocytes ou de fibroblastes.

9. Utilisation d'un textile non-tissé selon l'une des revendications 1 à 4 ou d'un matériau surfacique selon l'une des revendications 5 à 7 à titre de matériau de peuplement cellulaire.

10. Utilisation d'un textile non-tissé selon l'une des revendications 1 à 4 à titre de précurseur de produit alimentaire.

11. Utilisation d'un textile non-tissé selon l'une des revendications 1 à 4 ou d'un matériau surfacique selon l'une des revendications 5 à 7 pour la fabrication d'un médicament sous forme de dépôt.

12. Utilisation d'un textile non-tissé selon l'une des revendications 1 à 4 ou d'un matériau surfacique selon l'une des revendications 5 à 7 à titre de support pour un agent pharmaceutique actif.

13. Procédé pour la fabrication de textile en fibres non-tissé selon l'une des revendications 1 à 4, comprenant les opérations suivantes :
(a) préparation d'une solution de filature aqueuse comprenant un matériau à base de gélatine ;
(b) échauffement de la solution de filature à une température de filature ;
(c) traitement de la solution de filature chauffée dans un appareil de filature avec un rotor, de sorte que des fibres sont engendrées à partir du matériau à base de gélatine ; et
(d) capture des fibres engendrées avec formation d'un textile en fibres non-tissé présentant une pluralité de zones au niveau desquelles deux ou plusieurs fibres se transforment les unes dans les autres sans limite de phase,
**caractérisé en ce que** l'on utilise un rotor de filature qui présente des ouvertures de filature de tailles différentes, de sorte qu'il en résulte des fibres avec une épaisseur moyenne différente.

14. Procédé selon la revendication 13, **caractérisé en ce que** la solution de filature comprend une proportion de gélatine dans la plage d'environ 10 à environ 40 % en poids.
